# EUROPÄISCHE PATENTSCHRIFT

(11) **EP 3 338 519 B1**
(45) Veröffentlichungstag und Bekanntmachung des Hinweises auf die Patenterteilung: **14.01.2026**
(21) Anmeldenummer: 16758108.1
(22) Anmeldetag: 18.08.2016
(51) Int. Cl.: H05K 1/02, H05K 1/14, H05K 1/16

(54) **GEGENSTAND MIT EINER ELEKTRONISCHEN EINHEIT UND MIT LEITERSTRUKTUREN AUF EINER TRÄGERSTRUKTUR**
OBJECT HAVING AN ELECTRONIC UNIT AND CONDUCTOR STRUCTURES ON A CARRIER STRUCTURE
OBJET AVEC UNE UNITÉ ÉLECTRONIQUE ET AVEC DES STRUCTURES CONDUCTRICES SUR UNE STRUCTURE PORTEUSE

(30) Priorität: 21.08.2015 DE 102015113928
(43) Veröffentlichungstag der Anmeldung: 27.06.2018
(73) Patentinhaber: Schreiner Group GmbH & Co. KG, 85764 Oberschleissheim (DE)
(72) Erfinder: BECKER, Johannes, 85304 Ilmmünster (DE)
(74) Vertreter: Epping - Hermann - Fischer
(86) Internationale Anmeldenummer: PCT/EP2016/069600
(87) Internationale Veröffentlichungsnummer: WO 2017/032684

(56) Entgegenhaltungen:
- EP-A1- 1 416 581
- DE-A1- 102009 018 285
- DE-A1- 4 426 908
- DE-B4- 102009 018 285
- US-A- 5 466 892
- US-A1- 2010 085 258
- US-A1- 2013 230 755
- WAH Y C ET AL: "FOLDABLE Y-SHAPE FLEX", MOTOROLA TECHNICAL DEVELOPMENTS, MOTOROLA INC. SCHAUMBURG, ILLINOIS, US, vol. 32, 1 September 1997 (1997-09-01), pages 21/22, XP000741111, ISSN: 0887-5286
- SOLDNER K ET AL: "POST ASSEMBLY MULTI-LAYER INTERCONNECTS FOR FLEXIBLE CIRCUITS", MOTOROLA TECHNICAL DEVELOPMENTS, MOTOROLA INC. SCHAUMBURG, ILLINOIS, US, vol. 10, 1 March 1990 (1990-03-01), pages 19/20, XP000114622, ISSN: 0887-5286

## Beschreibung

Die Anmeldung betrifft einen Gegenstand mit einer elektronischen Einheit und mit Leiterstrukturen auf einer Trägerstruktur; insbesondere einen Gegenstand, der auf einer Trägerstruktur mindestens eine erste Leiterstruktur, zumindest eine elektronische Einheit und mindestens eine zweite Leiterstruktur aufweist, wobei die zweite Leiterstruktur von der ersten Leiterstruktur und/oder von der elektronischen Einheit galvanisch getrennt, aber daran elektrisch ankoppelbar ist.

US 5 466 892 A betrifft eine Schaltungsplatine und ein Verfahren zur Herstellung einer Schaltungsplatine mit Signal- und Rezeptorfeldern, die auf in Z-Richtung beabstandeten Schichten mit einer dazwischenliegenden dielektrischen Schicht angeordnet sind.

US 2013/230755 A1 betrifft ein flexibles Kabel mit einem in mindestens zwei Abschnitte unterteilten Substrat. Der erste Abschnitt enthält eine erste elektrisch leitende Bahn und der zweite Abschnitt weist eine zweite und dritte elektrisch leitende Bahn auf. Der erste Abschnitt ist auf dem zweiten Abschnitt angeordnet, um die erste elektrisch leitende Bahn mit der dritten elektrisch leitenden Bahn zu verbinden. Das resultierende flexible Kabel kann mit einem elektrischen Gerät verwendet werden.

SOLDNER K ET AL: "POST ASSEMBLY MULTI-LAYER INTERCONNECTS FOR FLEXIBLE CIRCUITS", MOTOROLA TECHNICAL DEVELOPMENTS, MOTOROLA INC. SCHAUMBURG, ILLINOIS, US, Bd. 10,1. März 1990 (1990-03-01), Seite 19/20, XP000114622, ISSN: 0887-5286 betrifft elektrische Schaltungen, bei denen Leiterbahnen auf derselben Seite eines flexiblen Trägermaterials erzeugt werden. Die elektrischen Komponenten werden dann mit konventionellen Techniken auf die Schaltung aufgebracht. Zusätzlich zum Aufbringen der Komponenten auf die Schaltung wird das Lötverfahren dazu verwendet, Lot auf Pads aufzubringen, die Verbindungsstellen zwischen den beiden unabhängigen Metamustern bilden.Nach dem Anbringen der Komponenten wird die flexible Schaltung auf sich selbst gefaltet, sodass ein Mehrschichtschaltkreiseffekt entsteht.

DE 10 2009 018285 A1 betrifft eine Blisterpackung, die aufweist: einen Blisterkörper mit Taschen zur portionsweisen Aufbewahrung und Entnahme von Medikamenten, Leiterbahnen, die relativ zu den Taschen so angeordnet sind, dass beim Entleeren einer Tasche jeweils eine zugeordnete Leiterbahn lokal durchtrennt wird, und einen RFID-Chip mit einem Halbleiterspeicher in dem Daten speicherbar sind, die eine Information darüber enthalten, ob und zu welchem Zeitpunkt die Leiterbahnen durchtrennt worden sind, wobei die Leiterbahnen und der RFID-Chip gegeneinander isoliert und voneinader entkoppelt sind und wobei der RFID-Chip ein passiver Chip ist, der für eine passive Energieversorgung und für eine passive Kommunikation ausgelegt ist.

In verschiedensten Gebieten der Technik sind Gegenstände im Einsatz, die eine oder mehrere auf einer Trägerstruktur montierte elektronische Einheiten aufweisen. Solche elektronischen Einheiten können beispielsweise aktive oder passive Bauelemente, beispielsweise Widerstände, Kondensatoren, Spule, sonstige elektrische oder elektronische Bauteile oder auch komplexere elektronischen Einheiten, beispielsweise Chips mit elektronischen bzw. mikroelektronischen Schaltungen sein. Eine elektronische Einheit kann auch eine Sensoreinheit sein, die beispielsweise ein Sensorelement zur Messung einer beliebigen physikalischen Größe bzw. eines Parameters und/oder seines Parameterwertes umfasst oder jedenfalls mit solch einem Sensorelement verbunden oder verbindbar ist. Diese Aufzählung exemplarischer, in Frage kommender elektronischer Einheiten ist nicht abschließend und ließe sich beliebig fortsetzen. Ferner kann eine elektronische Einheit auch eine Mehrzahl von elektrischen und/oder elektronischen Bauelementen, Chips und/oder sonstigen Bauteilen umfassen; beispielsweise können mehrere baulich getrennte, aber jeweils mit der Trägerstruktur einzeln verbundene elektronische Elemente gemeinsam eine elektronische Einheit bilden; entsprechend der Gesamtfunktion dieser Gruppe von Bauteilen.

Beispielhaft genannt seien hier Auswerteelektroniken für Feuchtemessungen bzw. Feuchtigkeitssensoren, Auswerteelektroniken zum Nachweis und/oder zur zeitlichen Erfassung von Unterbrechungen in Leiterbahnen, von Kurzschlüssen zwischen Leiterbahnen oder von temporären Veränderungen der Kapazität von Kondensatoren oder der Induktivität von Spulen; etwa in Abhängigkeit von äußeren Umgebungseinflüssen oder menschlichen Interaktionen. Weiterhin kommen als elektronische Einheiten Chips und/oder Transponder zur NFC-Kommunikation in Frage, beispielsweise RFID-Chips oder Chips zur Kommunikation über Bluetooth oder WLAN.

Solche Gegenstände weisen eine Trägerstruktur auf, auf der oder innerhalb derer außerdem Leiterstrukturen, insbesondere Leiterbahnen angeordnet sind.

Die meisten Gegenstände besitzen starre Trägerstrukturen, beispielsweise Printed Circuit Boards (PCB) oder sonstige, bestimmungsgemäß starre Trägerplatten, die beispielsweise mit einem Gehäuse verschraubbar sind. Es existieren jedoch auch Gegenstände mit flexiblen, d.h. biegsamen Trägerstrukturen, nämlich Gegenstände, bei denen die elektronische Einheit und/oder die Leiterstrukturen auf eine Folie aufgebracht sind.

Als Beispiel hierfür seien Feuchtigkeitssensoren für Windeln gegen Inkontinenz genannt, die Feuchtigkeit oder Nässe durch veränderte Kapazitäts- oder Induktivitätswerte oder durch elektrische Kurzschlüsse nachweisen können, bei denen aber eine starre Trägerstruktur wegen der Körperformen ungeeignet wäre.

Weiterhin existieren Blisterpackungen für Tabletten, Medikamentenkapseln oder sonstige Dosiseinheiten, die vom Patienten oder vom ärztlichen Personal beim Öffnen verbogen und lokal beschädigt werden, was als Unterbrechung der zugeordneten elektrischen Leitung, die der jeweiligen Tablette oder Kapsel und/oder der umgebenden Kavität zugeordnet ist, nachweisbar und auswertbar ist. Eine Unterbrechung einer Leiterbahn wird dabei als Entnahme bzw. Einnahme durch den Patienten interpretiert und zeitlich erfasst.

Des Weiteren existieren z.B. elektronische Siegel, die einen geschlossenen Stromkreis erzeugen und aufrechterhalten, wobei eine durch einen unbefugten Eingriff entstehende Zerstörung oder zeitweilige Unterbrechung des geschlossenen Stromkreises oder eine Veränderung von Kapazitäts- oder Induktivitätswerten elektronisch erfassbar und als Siegelbruch nachweisbar ist.

Solche und andere Gegenstände lassen sich grundsätzlich auch unter Verwendung einer Folie oder einer anderen flexiblen, d.h. biegsamen Trägerstruktur herstellen, allerdings ist die folienbasierte Herstellung solcher und anderer Gegenstände herkömmlich wenig effizient.

Wenn beispielsweise elektronische Einheiten oder Teilkomponenten davon auf eine Kunststofffolie gelötet werden, werden entweder Folien aus temperaturfestem Material, beispielsweise mit Arbeitstemperaturen oberhalb von 250° C benötigt, die aber den Herstellungsaufwand und die Materialkosten erhöhen. Alternativ können niedrigschmelzende Lote verwendet werden, die jedoch ebenfalls erheblich teurer sind. Das Auflöten oder Anlöten elektronischer Komponenten an flexible Kunststofffolien ist insbesondere dann wenig rationell, wenn auf ausgedehnten, d.h. großflächigen Folien nur relativ wenige Lötverbindungen innerhalb der Folienfläche zu fertigen sind oder die Lötstellen nur in einem oder in einigen wenigen Flächenbereichen herzustellen sind, die im Vergleich zur gesamten Folienfläche eher klein sind.

Anstelle von Lötverbindungen kann auch ein Leitkleber aufgebracht werden, um elektronische Einheiten leitend mit der Folie zu verbinden. Allerdings härten Leitkleber nur langsam aus, was wiederum den Materialdurchsatz des Herstellungsprozesses verlangsamt; auch für das Herstellen von Leitklebeverbindungen wird somit eine gewisse Zeit benötigt. Die für die Löt- oder Leitklebe-Verbindungen erforderliche Abkühl- oder Aushärtedauer ist unabhängig von der Foliengröße in sonstigen Flächenbereichen abseits der Löt- oder Leitklebeverbindungen. Gerade bei großen Folien mit wenigen elektrischen Kontaktierungsstellen wird die Fertigung, insbesondere die Bestückung und Montage elektronischer Komponenten und Einheiten schnell unwirtschaftlich.

Vorhandene Anlagen und Prozesse zum Bestücken mit elektronischen Komponenten bzw. zu deren Kontaktierung sind zudem im Durchsatz begrenzt. Bei vorgegebener Anzahl elektronischer Komponenten pro Exemplar des herzustellenden Gegenstandes ist beispielsweise die Geschwindigkeit zum Platzieren einer Komponente auf der Trägerstruktur durch die Herstellungsapparatur begrenzt; ferner ist das Volumen oder die Grundfläche in Bestückungs- und Kontaktierungsanlagen begrenzt und je nach Größe des Produkts schnell aufgebraucht, bevor das Material für die nächste Charge von Exemplaren des herzustellenden Gegenstandes zugeführt wird.

Es ist die Aufgabe der vorliegenden Anmeldung, einen Gegenstand bereitzustellen, der auf einer Trägerstruktur eine elektronische Einheit und Leiterstrukturen aufweist, der aber dennoch zu geringeren Gesamtkosten und niedrigerem Herstellungsaufwand herstellbar und somit kostengünstiger ist. Es ist ferner die Aufgabe der vorliegenden Anmeldung, ein geeignetes Herstellungsverfahren bereitzustellen.

Die Erfindung betrifft einen Gegenstand mit den Merkmalen des Anspruchs 1, einen Gegenstand mit den Merkmalen des Anspruchs 14 und ein Verfahren mit den Merkmalen des Anspruchs 16.

Einige exemplarische Ausführungsformen werden nachstehend mit Bezug auf die Figuren beschrieben. Es zeigen:
Figur 1 eine schematische Querschnittsansicht einer ersten exemplarischen Ausführungsform eines erfindungsgemäßen Gegenstandes,
Figur 2 eine schematische Draufsicht auf den Gegenstand gemäß Figur 1,
Figur 3 eine Draufsicht auf eine zweite Ausführungsform des Gegenstandes,
Figur 4 eine alternative Ausführungsform eines Gegenstandes mit je einer einzigen ersten und zweiten Leiterstruktur,
Figur 5 eine exemplarische Ausführungsform eines Gegenstandes mit induktiver statt kapazitiver Kopplung,
Figur 6 ein Ausführungsbeispiel eines Gegenstandes mit einer kapazitiven Brücke,
Figur 7 ein Ausführungsbeispiel eines als Blisterpackung ausgebildeten Gegenstandes,
Figur 8 eine schematische Draufsicht auf einen als elektronisches Siegel ausgebildeten Gegenstand,
Figur 9 das elektronische Siegel aus Figur 8 im zusammengelegten Zustand,
Figur 10 eine schematische Querschnittsansicht des geschlossenen elektronischen Siegels aus Figur 9,
Figur 11 eine weitere Ausführungsform eines elektronischen Siegels alternativ zu Figur 8 und
Figur 12 noch eine weitere Ausführungsform eines elektronischen Siegels alternativ zu den Figuren 8 und 11.

Erfindungsgemäß wird vorgeschlagen, anstelle einer Einzelfolie, die herkömmlich als Trägerstruktur für die elektronische Einheit und für Leiterbahnen dient, die Trägerstruktur zumindest bereichsweise, insbesondere in einem Teil ihrer Grundfläche als Schichtenstapel zu gestalten, d.h. als Schichtverbund, der mindestens zwei Folien oder Folienabschnitte oder zwei sonstige Trägerschichtbereiche umfasst, die aufeinander oder übereinander gestapelt sind; ggfs. mit einer dazwischen angeordneten Klebeschicht oder sonstigen Zwischenschicht. Dies erfordert bei den meisten der nachstehend noch im Einzelnen geschilderten Ausführungsformen zwar die Hinzufügung und Verarbeitung einer weiteren, zusätzlichen Folie oder Trägerschicht, weshalb für den Fachmann eine solche Lösung a priori wenig zielführend erscheint, wenn es darum geht, die Kosten und den Herstellungsaufwand zu verringern. Die zusätzliche Folie oder Trägerschicht ermöglicht es jedoch, die insgesamt erforderliche Folienfläche bzw. Grundfläche der Trägerstruktur in der Weise in Grundflächen zweier separater Trägerschichten und einen Überlappungsbereich, in dem beide Trägerschichten übereinander gestapelt sind, aufzuteilen, dass unter dem Gesichtspunkt der Durchsatzoptimierung bei der Verarbeitung und Bestückung der jeweiligen Einzelfolien dennoch ein letztendlicher Gewinn an Produktionszeit und/oder Materialdurchsatz entstehen kann. Beispielsweise lässt sich ausgehend von einer Gesamtgrundfläche, die herkömmlich durch eine einzige Folie ausgefüllt wird, die Grundfläche derjenigen Folie, die mit der elektronischen Einheit zu bestücken ist, soweit verkleinern, dass sich diese Folie in lateraler Richtung nur über die unmittelbare Umgebung der elektronischen Einheit erstreckt, jedoch weiter entfernt liegende Flächenbereiche nur durch die andere, zweite Folie bzw. Trägerschicht ausgefüllt werden. Die mit der elektronischen Einheit zu bestückende Folie braucht nicht einmal über die andere Folie hinauszuragen, sondern kann eine Teilfläche von ihr einnehmen, etwa einen Grundflächenbereich, in dem sie auf ihr gestapelt, beispielsweise mit ihr flächig verklebt ist.

Auf der anderen, zweiten Folie ist die zweite Leiterstruktur angeordnet, die beispielsweise eine zweite, an einen zweiten Kontaktanschluss der elektronischen Einheit angeschlossene Leiterbahn, eine Kondensatorplatte, eine Spule, eine Antenne, und/oder eine Elektrode umfassen kann. Die Leiterstrukturen auf den beiden Trägerschichten bzw. Folien können drucktechnisch, d.h. ohne Zuhilfenahme von Lötverbindungen oder Leitklebeverbindungen aufgebrachte oder anderweitig hergestellte Leiterstrukturen sein. Obwohl sie auf unterschiedlichen Folien bzw. Trägerschichten angeordnet sind, brauchen sie nicht leitend miteinander verbunden zu sein, sondern können galvanisch voneinander getrennt bleiben, soweit zumindest ein Wechselstromkreis zwischen ihnen erzeugbar ist. Daher braucht die mit der ersten Leiterstruktur und der elektronischen Einheit versehene Folie nicht mit der zweiten Folie, auf der die zweite Leiterstruktur angeordnet ist, verlötet oder durch Leitkleber in leitenden Kontakt gebracht zu werden. Fertigungstechnisch ist es ausreichend, nach Fertigstellung und Bestückung beider Folien bzw. Trägerschichten diese durch einen Klebe- oder Laminiervorgang aneinanderzufügen, wodurch der gebrauchsfertige Gegenstand entsteht.

Das Verlöten oder anderweitige Kontaktieren der elektronischen Einheit, insbesondere mit der kleineren von beiden Folien oder Trägerschichten, kann daher mit erhöhter Durchsatzrate und somit besonders effizient erfolgen. Zudem ist auch die andere, größere Folie, die vorher nur drucktechnischen Prozessen ausgesetzt wird, in größeren Stückzahlen pro Zeiteinheit herstellbar.

Die erste und die zweite Leiterstruktur können galvanisch voneinander getrennt, aber kapazitiv oder induktiv aneinander gekoppelt sein, beispielsweise durch auf beiden Folien angeordnete, einander zugeordnete Kondensatorplatten oder Spulen bzw. Spulenwindungen. Durch Anlegen oder Anregen einer Wechselspannung oder eines Wechselstroms entsteht dann eine kapazitive oder induktive Kopplung durch beide Folien und ggfs. eine dazwischen angeordnete Zwischenschicht oder Klebeschicht hindurch, ohne dass die Funktionsfähigkeit der elektronischen Einheit durch die bereichsweise doppelt verlegte Folie beeinträchtigt wäre.

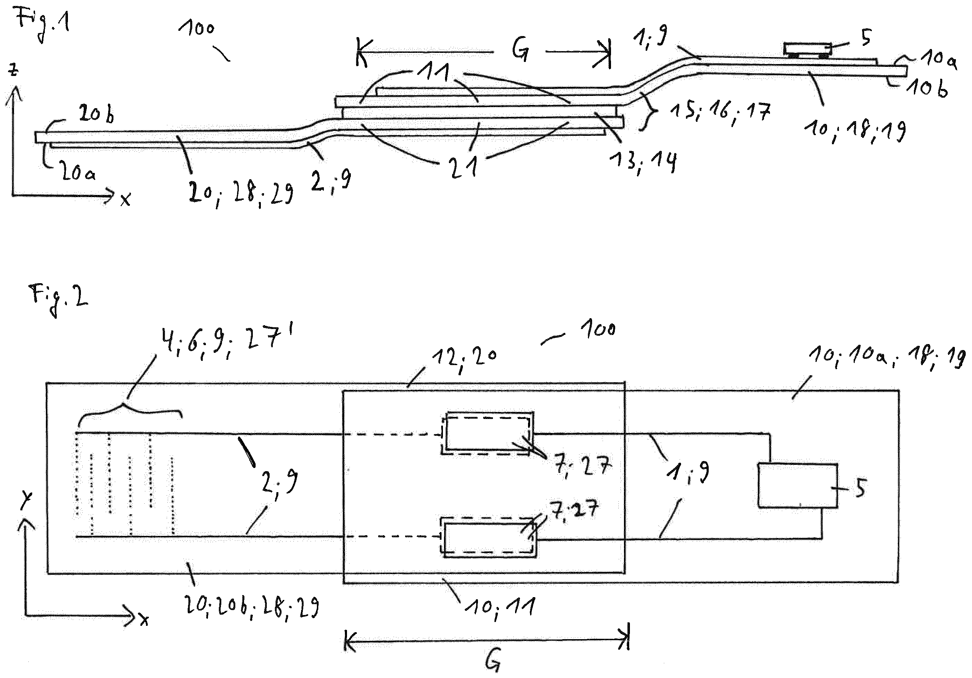

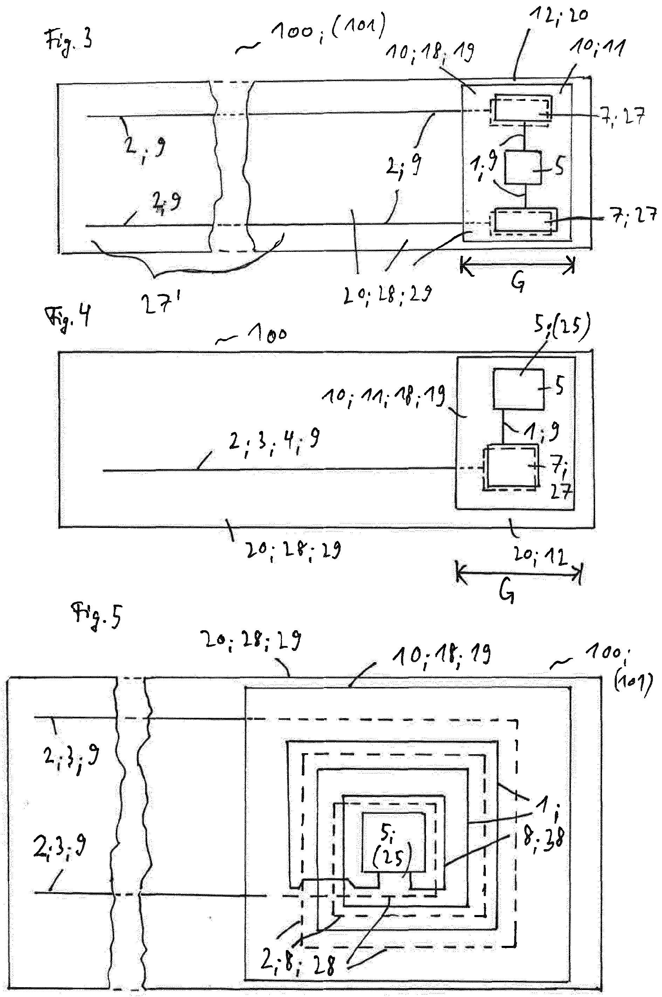

Die Kopplung beider Leiterstrukturen aneinander bzw. der zweiten Leiterstruktur an die erste Leiterstruktur und/oder an die elektronische Einheit erfolgt gemäß dieser Anmeldung nicht durch die elektronische Einheit selbst, sondern durch den Schichtenstapel, der die beiden Trägerschichten bzw. Trägerschichtbereiche (z.B. zwei Folien oder Folienabschnitte) mit der ersten und der zweiten Leiterstruktur umfasst. Die elektronische Einheit befindet sich außerhalb, d.h. auf die Grundfläche bezogen seitlich außerhalb desjenigen Teils der Grundfläche des Schichtenstapels, in welchem Grundflächenteil die elektrische Kopplung zwischen beiden Leiterstrukturen bzw. die Kopplung der zweiten Leiterstruktur an die erste Leiterstruktur und/oder an die elektronische Einheit stattfindet. Insbesondere kann die elektronische Einheit außerhalb der Grundfläche des Schichtenstapels angeordnet sein. Somit kann insbesondere der Schichtenstapel außerhalb der elektronischen Einheit angeordnet sein, d.h. die Grundfläche des Schichtenstapels und die Grundfläche der elektronischen Einheit überlappen einander nicht (Figuren 1 und 2). Alternativ kann (Figuren 3 bis 12) die Grundfläche des Schichtenstapels die Grundfläche der elektronischen Einheit umfassen, aber seitlich über diese hinausragen. In allen diesen Fällen erfolgt die elektrische Kopplung zwischen der ersten und der zweiten Leiterstruktur im einem nicht mit der elektronischen Einheit ausgefüllten (bzw. in einem nicht mit der elektronischen Einheit bedeckten) Teilbereich des Schichtenstapels bzw. seiner Grundfläche.

Insbesondere kann mit Hilfe des Schichtenstapels die zweite Leiterstruktur durch kapazitive oder induktive Kopplung mit der ersten Leiterstruktur verbunden werden. Beispielsweise kann, während das eine Teilstück oder Endstück der ersten Leiterbahn im Bereich des Schichtenstapels an ein entsprechendes Teilstück oder Endstück der zweiten Leiterbahn gekoppelt ist, ein entgegengesetztes Teilstück oder Endstück der ersten Leiterbahn mit der elektronischen Einheit verbunden sein. Auf diese Weise kann beispielsweise - wie auch in den nachfolgend diskutierten Figuren dargestellt ist - die zweite Leiterbahn über den Schichtenstapel und die erste Leiterbahn an die elektronische Einheit gekoppelt werden bzw. gekoppelt sein, obwohl die zweite Leiterbahn von ihnen galvanisch getrennt ist. Weiterhin können wie dargestellt insbesondere auch mehrere zweite Leiterbahnen über entsprechende Flächenbereiche des Schichtenstapels und über entsprechende erste Leiterbahnen an die elektronischen Einheit gekoppelt werden bzw. gekoppelt sein.

Konkret zeigt Figur 1 ein erstes Ausführungsbeispiel eines Gegenstandes 100 in schematischer Querschnittsansicht. Anstelle herkömmlich nur einer einzigen Folie bzw. Trägerschicht ist erfindungsgemäß sowohl eine erste Trägerschicht 10 als auch eine zweite Trägerschicht 20 vorgesehen; beide überlappen zumindest in einem Grundflächenbereich G, der einem Teilbereich der Grundfläche der Trägerstruktur 15 insgesamt entspricht. Die Trägerstruktur 15 umfasst daher beide Trägerschichten 10, 20; diese können wahlweise als Folien 19 bzw. 29, insbesondere Kunststofffolien, oder alternativ als anderweitige Materialfilms 18 bzw. 28, beispielsweise als Kunststofffilme (etwa Polyurethanfilme) oder als Lackfilme oder Lackschichten vorliegen. Materialfilme 18, 28, d.h. filmartige dünne Schichten aus geeignetem Material sind drucktechnisch auf beliebige Untergrundflächen aufdruckbar, von diesen wieder abziehbar und dann ähnlich wie Kunststofffolien einzeln handhabbar. Die zumindest eine zweite Leiterstruktur 2 kann beispielsweise drucktechnisch auf der zweiten Trägerschicht 2, d.h. auf der zweiten Folie 29 bzw. dem zweiten Materialfilm 28 aufgebracht sein, beispielsweise als Silberleitpaste oder sonstiger elektrisch leitfähiger Druckauftrag. In gleicher Weise kann die zumindest eine erste Leiterstruktur 1 auf die erste Trägerschicht aufgebracht werden. Beide Leiterstrukturen 1, 2 können außer Leiterbahnen 9 ferner Kondensatorplatten 7, Spulen und/oder Spulenwindungen und/oder Sensoren, insbesondere gedruckte, laminierte oder aufkaschierte Sensoren, umfassen.

Auf der ersten Trägerschicht 10 wird zusätzlich die mindestens eine elektronische Einheit 5 mittels Lötmaterial oder Leitklebematerial angebracht. Obwohl hierbei dieselben Verfahrensschritte anfallen wie beim Verlöten oder leitklebetechnischen Aufbringen der elektronischen Einheit auf eine Einzelfolie, kann die Grundfläche der ersten Folie im Vergleich zur Grundfläche der zweiten Folie so gering gewählt werden (siehe die nachfolgenden Figuren), dass eine viel größere Anzahl erster Folien gleichzeitig produziert, beispielsweise in einer Montageeinheit mit elektronischen Einheiten 5 bestückt wird, während eine Vielzahl von zweiten Folien vergleichsweise großer Grundfläche in einem davon unabhängigen Verfahrensschritt und in separaten Anlagen mit erhöhter Durchsatzrate gefertigt werden kann, einschließlich des Aufdruckens der zweiten Leiterstrukturen. Die Herstellung der größeren zweiten Folien wird somit durch das Bestücken der kleineren ersten Folien nicht behindert oder verzögert.

Es ist somit nicht erforderlich, die zweite Trägerschicht 20; 28; 29 einem Lötvorgang oder der Aufbringung von Leitklebermaterial zu unterziehen, wie es im Rahmen der Bestückung einer Einzelfolie mit elektronischen Komponenten oder Einheiten herkömmlich erforderlich ist. Vielmehr genügt es, die zu bestückende erste Folie 10 entsprechend der Grundfläche und/oder Position der elektronischen Einheit 5 zu dimensionieren und den übrigen, überwiegenden Teil der Trägerstruktur 15 insgesamt mit Hilfe der anderen, zweiten Trägerschicht 20, Folie 29 oder Materialbahn 28 allein zu gestalten. Lediglich in einem kleineren Grundflächenbereich G, in dem beide Trägerschichten 10, 20 miteinander überlappen, brauchen beide Trägerschichten 10, 20 vorgesehen und übereinander gestapelt und ggfs. aneinander oder an einer Klebe- oder sonstigen Zwischenschicht befestigt zu sein.

Optional kann die mit der elektronischen Einheit 5 bestückte Trägerschicht 10 sich zusätzlich auch bereichsweise über die Grundfläche der größeren Trägerschicht 20 hinaus erstrecken, wie in Figur 1 dargestellt ist. Dies ist jedoch nicht erforderlich; es genügt, in lediglich einem Teilbereich der Grundfläche der Trägerschicht 20 eine deutlich kleinere Trägerschicht 10 anzubringen, die aber bereits mit der elektronischen Einheit 5 bestückt ist. Dabei kann auch die Notwendigkeit längerer Leiterstrukturen 1 bzw. Leiterbahnen 9 auf dieser Trägerschicht 10 entfallen, wenn z.B. die elektronische Einheit 5 bereits im Bereich des durch den Schichtenstapel 16 gebildeten Folienverbunds bzw. Schichtverbunds positioniert ist.

Somit wird ein Gegenstand mit einer Trägerstruktur 15 bereitgestellt, die in einem Grundflächenbereich G, der zumindest eine Teilfläche der Grundfläche der Trägerstruktur 15 insgesamt umfasst, als Schichtenstapel 16 ausgebildet ist, wobei der Schichtenstapel 16 in dem Grundflächenbereich 15 einen ersten Trägerschichtbereich 11 und einen zweiten Trägerschichtbereich 12 umfasst. Der erste Trägerschichtbereich 11 ist ein Teilbereich der ersten Trägerschicht 10, bzw. der ersten Folie 19 oder des ersten Materialfilms 18, insbesondere ein Teilflächenbereich. Der zweite Trägerschichtbereich 12 ist gemäß Figur 1 ein damit überlappender Flächenbereich einer anderen, zweiten Trägerschicht 20, bzw. einer zweiten Folie 29 oder eines zweiten Materialfilms 28. Beide Teilschichtbereiche 11, 12 sind übereinander gestapelt, d.h. in einen Schichtenstapel 16 bzw. Schichtverbund 17 integriert. Zwischen ihnen ist vorzugsweise eine Zwischenschicht 14, etwa in Form einer Klebeschicht 13 oder Laminierschicht angeordnet, die aber optional ist und entfallen kann, beispielsweise wenn durch ein Gehäuse des Gegenstands beide Trägerschichtbereiche 11, 12 im Bereich der Grundfläche G aneinander gedrückt und/oder aneinander gehalten werden. Im Bereich des Schichtenstapels 16 bzw. der Grundfläche G ist ferner zumindest ein Teil der zweiten Leiterstruktur 2 und vorzugsweise auch ein Teil der ersten Leiterstruktur 1 angeordnet (dies gilt vorzugsweise für alle Figuren und sonstigen Ausführungsformen der Anmeldung), wenngleich diese Leiterstrukturen oder Teile von ihnen nicht die gesamte Grundfläche G des Schichtenstapels ausfüllen müssen.

Die elektronische Einheit 5 in Figur 1 und den nachfolgenden Figuren kann jede beliebige elektronische Einheit sein, die in den einleitenden Ausführungen dieser Anmeldung genannt wurde. Auch die erste Leiterstruktur 1 und/oder die zweite Leiterstruktur 2 kann wie in den einleitenden Bemerkungen geschildert ausgebildet sein; insbesondere als Leiterbahn 9, Antenne 3, Spule, Spulenwindung 8 und/oder als Kondensatorplatte 7. Alternativ können die Leiterstrukturen 1, 2 oder eine oder einige von ihnen als Sensorelement eines Bedienelements, beispielsweise einer Bedientaste realisiert sein. Hinsichtlich der Anzahl vorgesehener Leiterstrukturen können beispielsweise jeweils eine erste und eine zweite Leiterstruktur 1; 2 oder jeweils zwei erste und zwei zweite Leiterstrukturen 1; 2 (etwa wie in Figur 1) vorgesehen sein.

Je nach Funktion der elektronischen Einheit 5 und nach flächenmäßigem und/oder räumlichem Aufbau des Gegenstandes 100 können unterschiedlichste Gegenstände kostengünstiger und rationeller als herkömmlich hergestellt werden. So kann der in Figur 1 lediglich schematisch gezeigte Gegenstand 100 beispielsweise ein Feuchtigkeitssensor 101, etwa für Inkontinenzwindeln sein, alternativ aber auch eine Blisterpackung, ein elektronisches Siegel, eine Sensorvorrichtung für einen beliebigen Parameter, wie eingangs bereits ausgeführt, oder ein Transponder für RFID- oder sonstige NFC-Anwendungen, um nur einige Beispiele zu nennen.

Der in Figur 1 stellenweise gekrümmte Verlauf der ersten und zweiten Trägerschichtbereiche 11, 12 bzw. Trägerschichten 10, 20, wie er zumindest in Bereichen seitlich nahe der Grundfläche G des Überlappungsbereichs dargestellt ist, deutet an, dass diese Trägerschichtbereiche bzw. Trägerschichten gemäß einer ersten Ausführungsform flexibel, d.h. biegsam sein können und daher eine gebogene bzw. verbiegbare Form besitzen können. Es können beispielsweise beide Trägerschichtbereiche bzw. Trägerschichten biegsam sein.

Zweitens ist alternativ denkbar, dass lediglich eine der beiden Trägerschichten biegsam ist, beispielsweise diejenige, auf der die elektronische Einheit und die erste Leiterstruktur angeordnet sind, oder alternativ diejenige Trägerschicht, auf der die zweite Leiterstruktur angeordnet ist.

Ferner ist denkbar, dass die Trägerstruktur oder ihre beiden Trägerschichten oder eine von beiden Trägerschichten lediglich lokal, d.h. nur bereichsweise biegsam bzw. verformbar ist; etwa in einem Flächenbereich, in dem bestimmungsgemäß eine Falt- oder Knicklinie, eine lokale Verbiegung, insbesondere Umbiegung der jeweiligen Trägerschicht, oder eine sonstige Verformung ausbildbar sein soll. Eine solche, zumindest lokale Verformung lässt sich dafür nutzen, eine der beiden Leiterstrukturen an die elektronische Einheit und/oder an die andere Leiterstruktur heranzuführen und so einen geschlossenen Wechselstromkreis zu ermöglichen. Beispielsweise kann der erste Trägerschichtbereich und/oder der zweite Trägerschichtbereich oder ein Teilflächenabschnitt davon biegsam sein, etwa um zumindest einen der Trägerschichtbereiche umzubiegen, umzuklappen oder in sonstiger Weise an den anderen Trägerschichtbereich oder einen Teilflächenabschnitt von ihm heranzuführen.

Bei solchen Ausführungsformen, bei denen nicht die gesamte Trägerstruktur biegsam ist, können diejenigen Trägerschichten oder Trägerschichtbereiche, die nicht biegsam sind oder jedenfalls nicht ohne Weiteres zum Verbiegen vorbestimmt sind, starr sein, eine größere Schichtdicke besitzen oder eine geringere Elastizität und/oder Biegsamkeit besitzen als diejenigen Trägerschichten oder Trägerschichtbereichen, die zumindest lokal zum Verbiegen oder anderweitigen Verformen bestimmt sind.

Ferner ist denkbar, dass lediglich die Leiterstrukturen oder zumindest eine von ihnen biegsam ist, entweder vollflächig oder zumindest lokal. Es ist beispielsweise denkbar, dass eine Leiterstruktur, die zwar an einer der beiden Trägerschichten befestigt ist, aber nicht vollflächig, d.h. nicht mit ihrer gesamten Fläche bzw. Unterseite, sondern nur teilflächig auf der Trägerschicht aufliegt, von der Trägerschicht lokal abgehoben wird, um sie an die jeweils andere Leiterstruktur und/oder an die elektronische Einheit heranzuführen.

Je nachdem, welche der obigen Ausführungsformen realisiert ist, können der Gegenstand, seine Trägerstruktur, seine Trägerschichten oder Trägerschichtbereiche und/oder seine Leiterstrukturen, soweit sie vollständig oder zumindest lokal biegsam sind, an einen anderen Körper angeschmiegt sein, beispielsweise um Orte zu erreichen, an die die elektronische Einheit 5 selbst nicht oder nicht nahe genug heranführbar ist.

Die Trägerschichten 10, 20 können insbesondere Folien 19, 29 oder Materialfilme 18, 28 aus geeigneten Kunststoffen oder anderen, insbesondere biegsamen Materialien sein. Optional ist auch der im Überlappungsbereich beider Trägerschichten 10, 20 gebildete Schichtenstapel 16 bzw. Schichtverbund 17 ebenfalls biegsam.

Figur 2 zeigt eine schematische Draufsicht auf den Gegenstand aus Figur 1. Unterhalb eines Teils der ersten Trägerschicht 10 ist ein Teil der zweiten Trägerschicht 20 angeordnet. Im Überlappungsbereich mit der Grundfläche G umfassen die ersten und zweiten Leiterstrukturen 1, 2 jeweils Leiterbahnabschnitte von Leiterbahnen 9 sowie einander zugeordnete Kondensatorplatten 7. Die dadurch gebildeten Kondensatoren 27 ermöglichen eine Kopplung zwischen den Leiterstrukturen 1, 2 beider Trägerschichten 10, 20 durch einen Wechselstromkreis; etwa durch Anlegen oder Induzieren einer Wechselspannung an den durch die ersten und zweiten Leiterstrukturen gebildeten (Wechsel-)Stromkreis. Die ersten Leiterstrukturen 1 sind mit einem Ende an die elektronische Einheit 5 und mit dem anderen Ende an eine jeweilige Kondensatorplatte 7 angeschlossen. Überlappend mit diesen, d.h. unter ihnen und ggfs. durch eine Zwischenschicht 14 bzw. Klebeschicht 13 sowie die Schichtdicken einer oder beider Trägerschichten 10, 20 getrennt, befinden sich zugeordnete Kondensatorplatten 7 der zweiten Leiterstrukturen 2. Die zweiten Leiterstrukturen 2 umfassen ferner weitere Leiterbahnen 9, die entweder linienförmig mit offenem Ende (links in Figur 2) verlaufen können oder, alternativ, wie in Figur 2 durch gepunktete Linien dargestellt, eine Interdigitalstruktur umfassen können. Aber auch ohne eine solche Interdigitalstruktur bildet das Paar von zweiten Leiterstrukturen 2 einen Kondensator, der beispielsweise zur Feuchtigkeitsmessung in der Umgebungsluft einsetzbar ist. Länge und/oder gegenseitiger Abstand der beiden zweiten Leiterstrukturen 2 voneinander auf der zweiten Trägerschicht 20 werden geeignet gewählt. Die zweiten Leiterstrukturen 2; 9 oder jedenfalls Endstücke oder Teilstücke von ihnen können weiterhin ein Sensorelement 4 für einen beliebigen Parameter oder einen Berührungssensor, etwa einen kapazitiven oder induktiven Berührungssensor für ein Bedienfeld oder ein Bedienelement, etwa für eine Bedientaste umfassen. Der Wechselstromkreis zum Betrieb des Gegenstands 100 kann durch die elektronische Einheit 5 selbst erzeugbar sein oder von außen induziert werden, etwa durch RFID- oder anderweitige drahtlose Übertragung.

Figur 3 zeigt eine Draufsicht auf eine andere Ausführungsform eines Gegenstands. Im Gegensatz zu Figur 2 reicht in lateraler Richtung die erste Trägerschicht 10 bzw. Folie 19 oder Materialbahn 18 nicht über die zweite 20; 28; 29 hinaus, sondern verläuft vollständig innerhalb eines Teilflächenbereichs von dieser, beispielsweise an einem endseitigen Rand (rechts in Figur 3). Im Übrigen wird hinsichtlich Figur 3 sowie aller nachfolgenden Figuren erneut auf die Ausführungen zu den Figuren 1 und 2 sowie auf einleitenden Bemerkungen der Beschreibung verwiesen.

In Figur 3 ist der Kondensator 27' in Form zweier paralleler, unverzweigter Leiterbahnen 2; 9 ausgebildet. Sofern der Gegenstand 100 ein Gehäuse (nicht dargestellt) umfasst, braucht dieses nur den Überlappungsbereich beider Trägerschichten 10, 20 zu umschließen; dies gilt ebenso für alle übrigen Figuren und sonstigen Ausführungsformen der Anmeldung. In Figur 3 genügt ein Gehäuse, das randseitig (rechts in Figur 3) angebracht ist, wohingegen der größte Flächenanteil der ersten Trägerschicht 10 bzw. Folie aus dem Gehäuse herausragen kann.

Figur 4 zeigt eine alternative Ausführungsform eines Gegenstandes 100, der nur eine einzige, zusammenhängende erste Leiterstruktur 1 und nur eine einzige, zusammenhängende zweite Leiterstruktur 2 auf der jeweiligen Folie aufweist. Die Kopplung zwischen beiden Folien bzw. ihren Leiterstrukturen erfolgt wieder durch den Kondensator 27. Die zweite Leiterstruktur umfasst außer einer der Kondensatorplatten 7 hauptsächlich eine unverzweigte Leiterbahn 9, deren Länge im wesentlichen die Länge der zweiten Folie 20 in Richtung einer ersten lateralen Richtung x (vgl. mit Figur 1 und 2) bestimmt. Bei der Ausführungsform der Figur 4 bildet die zweite Leiterstruktur 2; 9 eine Antenne 3 oder ein sonstiges Sensorelement 4; die Schließung des Wechselstromkreises erfolgt über Masse bzw. Erde und somit wiederum drahtlos. Die elektronische Einheit 5 kann beispielsweise einen RFID-Chip 25, einen sonstigen NFC-Chip und/oder einen anderweitigen Transponder umfassen.

Figur 5 zeigt eine weitere Ausführungsform mit einer induktiven statt kapazitiven Kopplung zwischen den Leiterstrukturen beider Folien. Die an zwei Anschlüsse der elektronischen Einheit 5 angeschlossene erste Leiterstruktur 1 bildet eine Spule 38 mit einer oder mehreren Spulenwindungen 8 und umläuft vorzugsweise die elektronische Einheit 5 in lateraler Richtung. Überlappend mit ihr befindet sich eine zugeordnete Spule oder Spulenwindung an oder auf der zweiten Trägerschicht 20 (gestrichelt dargestellt, da durch die erste Trägerschicht 10 verdeckt), die einen Bestandteil der zweiten Leiterstruktur 2 bildet und an ihren beiden Enden in zwei außerhalb des Überlappungsbereichs befindliche, längliche Leiterbahnenabschnitte 9 mündet, die ebenfalls zur zweiten Leiterstruktur 2 gehören. Die zweite Leiterstruktur 2, die somit induktiv an die erste Leiterstruktur 1 gekoppelt oder koppelbar ist, kann wiederum einen Sensor zum Nachweis irgendeines Parameters, beispielsweise der Feuchtigkeit bilden, alternativ einen sonstigen Sensor, etwa einen Berührungssensor oder eine Antenne umfassen. Während die zweite Leiterstruktur 2 in Figur 4 als Monopolantenne realisiert ist, kann sie alternativ auch als Dipolantenne oder als eine sonstige Antenne realisiert sein.

Bei allen Figuren und Ausführungsformen dieser Anmeldung können die ersten und/oder zweiten Leiterstrukturen 1, 2 statt auf der Außenseite 10a, 20a der jeweiligen Trägerschicht 10, 20 alternativ auch auf deren Innenseite 10b, 20b angeordnet sein. Somit können gegenüber dem Schichtaufbau, wie er in Figur 1 exemplarisch dargestellt ist, die ersten Leiterstrukturen 1 oder die zweiten Leiterstrukturen 2 oder beide von ihnen zwischen den einander zugewandten Hauptflächen der beiden Trägerschichten 10, 20 angeordnet sein. Jedoch auch dann verlaufen sie so und/oder sind sie so durch eine Klebe- oder anderweitige Zwischenschicht voneinander getrennt, dass zwischen ihnen kein Strom, insbesondere kein Gleichstrom fließen kann, d.h. beide Leiterstrukturen sind vorzugsweise als voneinander galvanisch voneinander getrennte Leiterstrukturen dimensioniert und ausgelegt.

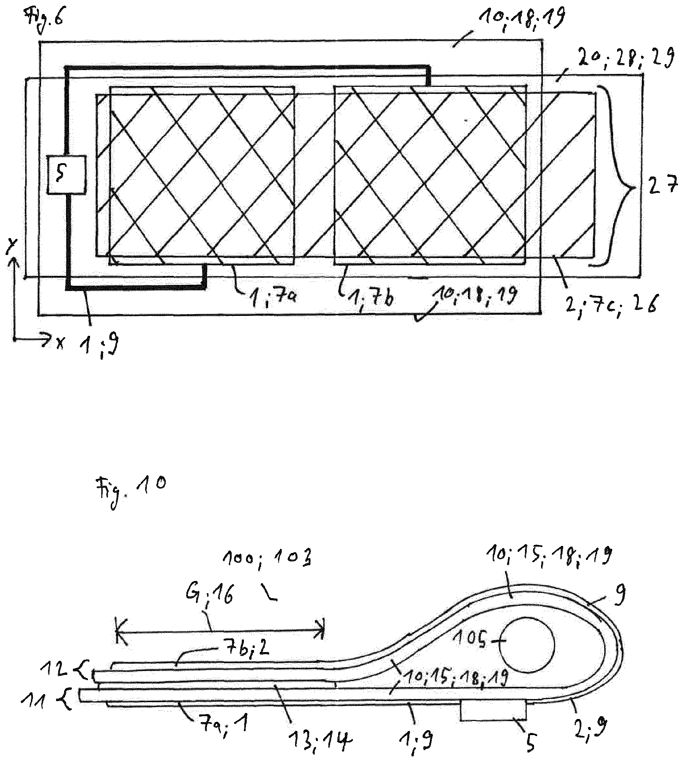

Figur 6 zeigt eine weitere Ausführungsform, bei der auf der ersten Trägerschicht 10 zwei relativ großflächige Kondensatorplatten 7a, 7b über entsprechende Leiterbahnen 1; 9 mit der elektronischen Einheit 5 verbunden sind. Auf der zweiten Trägerschicht 20 hingegen bildet die zweite Leiterstruktur 2 eine dritte Kondensatorplatte 7c, die als kapazitive Brücke 26 dient; sie überbrückt die seitliche Distanz zwischen beiden Kondensatorplatten 7a, 7b der beiden ersten Leiterstrukturen 1. Figur 6 ist ferner ein Beispiel für eine Ausführungsform, bei der zwei erste Leiterbahnen 1; 7a, 7b, aber nur eine einzige zweite Leiterbahn 2; 7c vorliegen. Der aus den ersten und zweiten Leiterbahnen 1, 2 gebildete Kondensator 27 besitzt eine Kapazität, die sich aus den Teilkapazitäten des Schichtaufbaus im Überlappungsbereich, nämlich einmal zwischen den Kondensatorplatten 7a und 7c und einmal zwischen den Platten 7c und 7b zusammensetzt. Die elektrisch isolierte Kondensatorplatte 7c besitzt näherungsweise die doppelte Kondensatorfläche wie jede der übrigen Kondensatorplatten 7a, 7b. Der Abstand zwischen den Kondensatorplatten 7a und 7b kann geeignet groß gewählt werden, um eine Störkapazität zwischen ihnen zu reduzieren. Ebenso wie in den übrigen Figuren sind die Abmessungen, Überlappungsbereiche und Dimensionierungen sowie Größenverhältnisse der Trägerschichten 10, 20 bzw. Folien 19, 29 oder Materialfilme 18, 28, d.h. lediglich beispielhaft und nicht maßstäblich.

Der Gegenstand 100 gemäß Figur 6, aber ebenso auch der Figuren 1 bis 5, ist beispielsweise als Sensor zur Messung der Feuchtigkeit der Umgebungsatmosphäre oder für einen sonstigen Sensor ausbildbar. Als Zwischenschicht 14 zwischen beiden Trägerschichten 10, 20 wird dann vorzugsweise ein Material verwendet, dessen Schichtdicke unabhängig von der Luftfeuchtigkeit oder einem sonstigen nachzuweisenden oder zu messenden oder zu überwachenden Parameter ist. Alternativ jedoch kann auch ein Material eingesetzt werden, dessen Schichtdicke oder deren sonstige Materialeigenschaft (etwa Polarisation, Dielektrizitätskonstante, Magnetisierung oder dergleichen) sich in Abhängigkeit von der Luftfeuchtigkeit oder einem sonstigen Messparameter verändert. Der Gegenstand der Anmeldung, insbesondere in der Ausführungsform gemäß Figur 6, eignet sich insbesondere für Messsensoren mit feuchtigkeitsabhängiger bzw. parameterabhängiger und somit veränderlicher Schichtdicke der Zwischenschicht 14; 13, d.h. für Messsensoren mit zumindest einer Zwischenschicht, deren Schichtdicke sich in Abhängigkeit von physikalischen und/oder chemischen Parametern ändert. Er eignet sich ebenso für Messsensoren mit zumindest einer Zwischenschicht, deren Dielektrizitätskonstante sich in Abhängigkeit von physikalischen und/oder chemischen Parametern ändert.

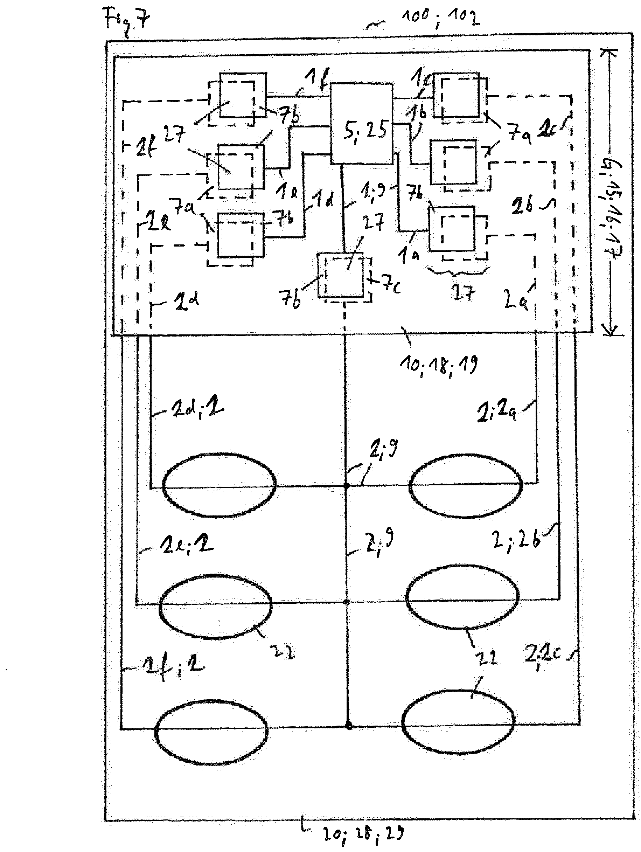

Figur 7 zeigt eine alternative Ausführungsform, bei der der Gegenstand 100 als Blisterpackung 102 oder jedenfalls als ein Teil von ihr ausgebildet ist. Die Blisterpackung 102 besitzt eine Plastikfolie, die bereichsweise zu Kavitäten 22 geformt ist, in welche die Tabletten, Kapseln oder sonstigen Medikamenteinheiten eingelegt sind, sowie eine Deckfolie zum Verschließen der Kavitäten. Die Deckfolie ist erfindungsgemäß zumindest bereichsweise als Doppelfolie, umfassend die beiden Trägerschichten 10, 20, ausgebildet. Der Gegenstand 100 kann somit eine Deckfolie einer Blisterpackung sein. Die elektronische Einheit 5, die einen RFID-Chip 25 oder einen sonstigen NFC-Transponder (ggfs. mit Antenne; nicht dargestellt) umfassen kann, ist auf der ersten Trägerschicht 10 montiert, die den Flächenbereich der Kavitäten aber nicht zu überlappen braucht. Zumindest die zweite Trägerschicht 20 jedoch überdeckt und verschließt die mit Tabletten oder Kapseln gefüllten Kavitäten 22 und erstreckt sich auch über deren Umgebungsbereiche. Die zweite Leiterstruktur 2; 9 umfasst Leiterbahnen 2a bis 2f, die jeweils über einzelne Kavitäten 22 hinweg laufen und beim Entnehmen der jeweiligen Tabletten unterbrochen, d.h. zerstört werden. Bei einer Anzahl von N Kavitäten (hier N = 6) sind somit N (also hier sechs) einzelne zweite Leiterstrukturen 2 vorgesehen, die von einer zusätzlichen weiteren Anschlussleitung 2; 9 abgezweigt sind, die beispielsweise gemäß Figur 7 die Abzweigungs- oder Knotenpunkte miteinander verbindet und über einen eigenen Kondensator (mit den Kondensatorplatten 7b und 7c) und über eine erste Leitung 1; 9 galvanisch an einen ersten Anschluss der elektronischen Einheit 5 auf der ersten Trägerschicht 10 gekoppelt ist. Die übrigen zweiten Leiterbahnen 2a bis 2f; 9 sind jeweils über weitere Kondensatoren (mit den Kondensatorplatten 7a und 7b) und über weitere erste Leiterstrukturen 1a bis 1f an weitere Anschlüsse der elektronischen Einheit 5 angeschlossen. Somit sind für N Kavitäten insgesamt N+1 erste bzw. zweite Leitungszweige und daher auch N+1 Anschlüsse an der elektronischen Einheit 5 erforderlich. Mit ihnen ist die Entnahme der jeweiligen Tablette und/oder der Zeitpunkt der Entnahme jeweils individuell erfassbar, speicherbar und/oder auswertbar. Im Übrigen ist die Blisterpackung aus Figur 7 mit jeder herkömmlichen Blisterpackung kombinierbar; bezüglich der sonstigen Einzemerkmale wird auf die vorigen Figuren verweisen. Die zweite Trägerschicht 20 überlappt mit der ersten Trägerschicht 10 zumindest in einem Randbereich seitlich außerhalb der Kavitäten, ist aber auch mit der stabileren Folie verbunden, welche zu Kavitäten 22 ausgeformt ist und hierfür eine höhere mechanische Festigkeit, Steifigkeit und/oder Schichtdicke besitzt als die Trägerschichten 10, 20.

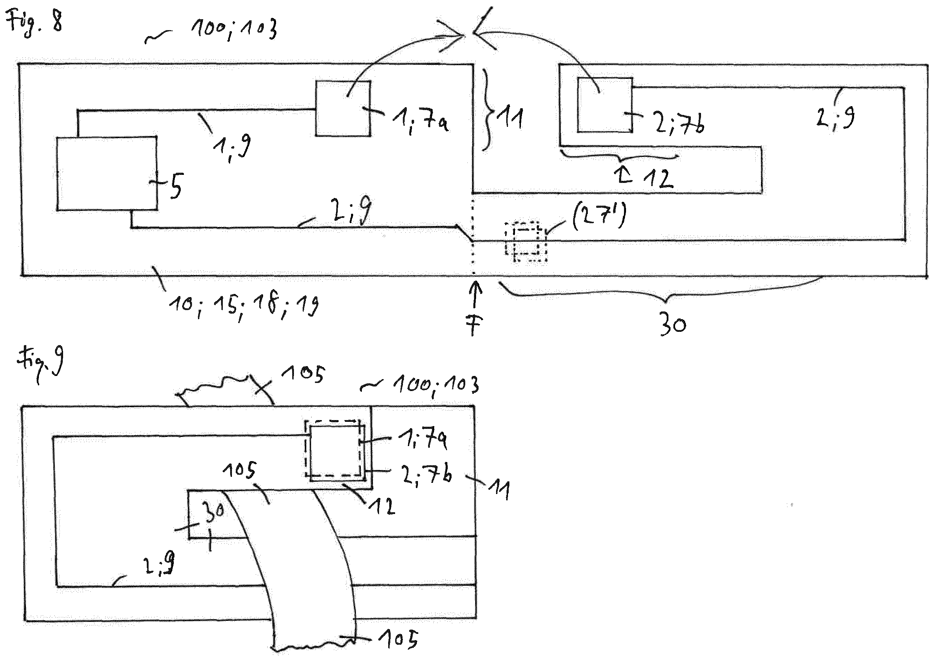

Figur 8 zeigt eine schematische Draufsicht auf einen als elektronisches Siegel 103 ausgebildeten Gegenstand 100. Der Gegenstand 100 weist zwei Leiterstrukturen 1, 2 und eine dazwischen geschaltete elektronische Einheit 5 auf. Zumindest eine der beiden Leiterstrukturen 1, 2 umfasst eine Leiterbahn 9, die überwiegend außerhalb der elektronischen Einheit 5 verläuft; gemäß Figur 8 ist dies für beide Leiterbahnen 1, 2 der Fall. Jede Leiterbahn 1, 2 kann zusätzlich noch eine Kondensatorelektrode oder Kondensatorplatte 7a, 7b umfassen oder alternativ eine Spulenwindung oder Spule (nicht dargestellt). Die beiden Leiterstrukturen 1, 2 sind dafür bestimmt, dass Endstücke oder zumindest Teilstücke von ihnen miteinander koppelbar sind, um einen geschlossenen Wechselstromkreis zu ermöglichen, spätestens wenn sie aneinander befestigt oder herangeführt werden. Die Grundfläche der Trägerstruktur 15 is so gestaltet, dass sie ein zu versiegelndes Objekt 105 (Figur 9) oder einen Teil davon umschließen und elektronisch sichern, d.h. versiegeln kann. Hierzu besitzt das elektronische Siegel 103 aus Figur 8 eine Trägerstruktur 15 aus nur einer einzigen Trägerschicht 10 (oder alternativ mit zumindest einer Trägerschicht 10), etwa einer Folie 19 oder eines Materialfilms 18. Gemäß Figur 8 sind beide Leiterstrukturen 1, 2 auf dieser Trägerschicht 10 angeordnet, entweder auf derselben Folienfläche oder auf entgegengesetzten Flächen. Die Grundfläche der Trägerschicht 10 bzw. Trägerstruktur 15 ist so gewählt, dass die Trägerschicht 10 bzw. Trägerstruktur 15 faltbar, knickbar, verbiegbar oder in sonstiger Weise zusammenlegbar oder schließbar ist, dass die beiden Trägerschichtbereiche 11, 12 aneinander heranführbar und/oder miteinander verklebbar sind, um einen Schichtverbund oder Schichtenstapel 16 zu bilden. Beispielsweise sind zwei jeweilige Endstücke und/oder Teilstücke der beiden Leiterstrukturen 1, 2 aneinander herangeführbar, um eine Kondensator oder eine Doppelspule zu bilden, wobei die Kapazität oder Induktivität oder eine sonstige Übertragungseigenschaft durch die elektronische Einheit 5 perodisch, zeitkontinuierlich und/oder über einen Zeitraum dauerhaft überwacht werden kann, um einen potenziellen Siegelbruch zu detektieren.

In Figur 8 gehören beide Trägerschichtbereiche 11, 12 derselben Folie 19 bzw. Trägerschicht 10 an; sie sind durch den Verbindungsbereich 30 miteinander verbunden und werden beim Zusammenlegen der Trägerstruktur 15 aufeinander gestapelt. Die elektronische Einheit 5 kann auf einem der beiden Trägerschichtbereiche 11, 12 oder auf dem Verbindungsbereich 30 angeordnet sein. Ferner verläuft mindestens eine der Leiterstrukturen 1, 2 im Verbindungsbereich 30 oder durch den Verbindungsbereich 30 hindurch, etwa zwischen der optionalen Falt- oder Knicklinie F und dem davon beabstandeten zweiten Trägerschichtbereich 12 in Figur 8.

Beide Leiterstrukturen 1, 2 können wahlweise leitend miteinander verbunden oder galvanisch voneinander getrennt sein. Die galvanische Trennung kann beispielsweise durch die elektronische Einheit 5 selbst, d.h. innerhalb von ihr erfolgen. Alternativ kann eine der Leiterstrukturen 1, 2 oder können beide von ihnen lokal durch einen Kondensator 27' oder eine Doppelspule unterbrochen sein. Die beiden Kondensatorplatten 7a, 7b oder Spulen liegen auf entgegengesetzten Hauptflächen der Trägerstruktur 15 bzw. Trägerschicht.

Figur 9 zeigt das elektronische Siegel aus Figur 8 im zusammengelegten und somit versiegelten Zustand. Dabei wurde der in Figur 8 rechts von der Faltlinie F befindliche Teil des Siegels 103 umgeklappt und auf den links von der Faltlinie F angeordneten Teil des elektronischen Siegels 103 gelegt. Dabei kommt ein Endstück oder Teilstück des zweiten Trägerschichtbereichs 12, insbesondere ein Endstück oder Teilstück der zweiten Leiterststruktur 2 (hier umfassend zumindest eine Kondensatorplatte 7b oder alternativ Spule oder Spulenwindung) auf oder über einen zugeordnetem Endstück oder Teilstück des ersten Trägerschichtbereichs 11 bzw. einem End- oder Teilstück der ersten Leiterstruktur 1 (hier umfassend eine Kondensatorplatte 7a, alternativ Spule oder Spulenwindung) zu liegen. Durch das Verbiegen, Falten, Knicken, Zusammenlegen oder anderweitiges Verschließen der Trägerstruktur 15 bzw. Trägerschicht 10 das elektronische Siegel 103 geschlossen, wobei noch ein Wechselstromkreis aktiviert wird, der zwischen beiden Trägerschichtbereichen 11, 12 (bzw. End- oder Teilstücken der beiden Leiterstrukturen 1, 2) auch durch den Schichtenstapel fließen kann.

Vor dem Schließen des Siegels 103 wurde damit ein zu versiegelndes Objekt 105 umschlossen (Figur 9), beispielsweise durch Herumführen des Verbindungsbereichs 30 um einen Teil des Objekts 105. Sobald der Wechselstromkreis geschlossen, d.h. die elektronische Einheit 5 und/oder das elektronische Siegel 103 aktiviert ist, wird jede Unterbrechung des Wechselstromkreises oder Veränderung der Kapazität oder Induktivität zwischen beiden Leiterstrukturen 1, 2 in dem Schichtenstapel 16 erfasst und als Siegelbruch interpretiert. Zudem kann der Siegelbruch auch durch ergänzende optische Anzeigen (z. B. Void-Effekt) oder durch mechanische Zerstörung (z. B. durch irreversible Verklebung) angezeigt werden
Figur 10 zeigt eine schematische Querschnittsansicht des geschlossenen elektronischen Siegels 103 aus Figur 9. Dabei sind beide Trägerschichtbereiche 11, 12 der Trägerstruktur 15 aufeinander gestapelt und bilden somit einen Schichtenstapel 16.

Gemäß den Figuren 8 bis 10 ist nicht nur der erste Trägerschichtbereich 11, sondern auch der zweite Trägerschichtbereich 12 ein Trägerschichtbereich der ersten Trägerschicht 10. Somit ist ein Schichtenstapel 16 im Bereich der Grundflächenbereich G mit Hilfe nur einer einzigen Trägerschicht 10, d.h. einer einlagigen Trägerstruktur 15 ausbildbar. Optional kann eine Klebeschicht 13 oder sonstige Zwischenschicht 14 enthalten sein. Die Position der elektronischen Einheit 5 in den Figuren 8 bis 10 ist lediglich exemplarisch. Alternativ kann sie auch dort angeordnet sein, wo in Figur 8 eine der Kondensatorplatten 7a oder 7b dargestellt ist. Eine der Leiterbahnen kann sogar vollständig in die elektronische Einheit 5 integriert oder vollständig über oder unter ihr angeordnet sein. Des Weiteren können die Leiterstrukturen 1, 2 oder eine von ihnen Durchkontaktierungen durch die Trägerschicht 10 hindurch aufweisen, beispielsweise am Ort des optional dargestellten Kondensators 27' in Figur 8. Somit kann sich zumindest eine der Leiterstrukturen abschnittweise auf jeder der beiden Hauptflächen der Trägerstruktur 15 bzw. Trägerschicht 10 befinden. Der Aufbau, die Geometrie und die Proportionen der Gegenstände, die in den Figuren dieser Anmeldung dargestellt ist, sind im Übrigen lediglich exemplarisch.

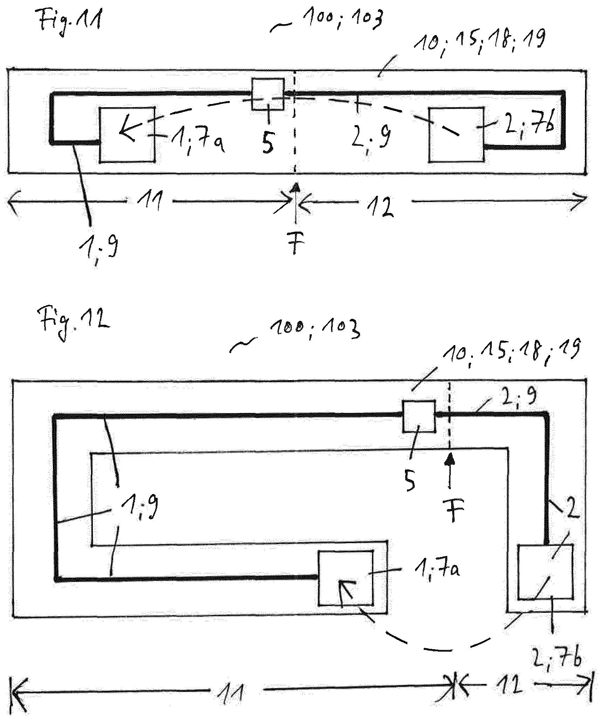

Während gemäß Figur 10 das elektronische Siegel aus Figur 8 so zusammengelegt oder zusammengeführt ist, dass sich die beiden Leiterstrukturen im Bereich der Schichtenstapels 16 an voneinander abgewandten Flächen bzw. Außenseiten der beiden Trägerschichtbereiche 11, 12 befinden, kann das elektronische Siegel 103 aus Figur 8 (und gleichermaßen dasjenige der Figuren 11 und 12; siehe unten) alternativ auch so zusammengelegt oder zusammengeführt sein, dass sich die beiden Leiterstrukturen im Bereich der Schichtenstapels 16 an den einander zugewandten Flächen bzw. Innenseiten der beiden Trägerschichtbereiche 11, 12 befinden.

Ferner kann das elektronische Siegel 103 auch so zusammengeführt werden bzw. so zu einer geschlossenen Schlaufe zusammengeführt sein, dass im Bereich der Schichtenstapels 16 eine der beiden Leiterstrukturen auf der Innenseite - bezogen auf die Anordnung innerhalb des Schichtenstapels 16 - des jeweiligen Trägerschichtbereichs angeordnet ist, wohingegen die andere Leiterstruktur auf der Außenseite des (anderen) Trägerschichtbereichs angeordnet ist; dann ist genau eine von beiden Leiterstrukturen der Klebeschicht zugewandt. Bei dieser Ausführungsform kann die Trägerschicht beim Zusammenführen im Übergangs- oder Verbindungsbereich zwischen beiden Trägerschichtbereichen zusätzlich um 180° verdreht werden.

Die Figuren 11 und 12 zeigen zu Figur 8 alternative Ausführungsformen zusammenklappbarer bzw. zusamenfaltbarer elektronischer Siegel 103, bei denen insbesondere die Flächenaufteilung der Trägerschicht 10 unterschiedlich gestaltet ist. Wiederum wird durch das Umschließen eines Objekts 105, wenn es in der Nähe der Knicklinie F an die Trägerschicht 10 herangeführt wird und beidseitig von den mit den Leiterstrukturen 1 und 2 bzw. Kondensatorplatten 7a, 7b versehenen Trägerschichtbereichen 11, 12 umschlossen wird, ein geschlossener Wechselstromkreis um das Objekt 105 herum ermöglicht; ähnlich wie in Figur 9 und 10 dargestellt.

Die in den Figuren 8, 11 und 12 gezeigten Flächengestaltungen und Flächenaufteilungen sind lediglich exemplarisch.

### Bezugszeichenliste

- 1: erste Leiterstruktur
- 2: zweite Leiterstruktur
- 2a-2f: Leiterbahnen
- 3: Antenne
- 4: Sensorelement
- 5: elektronische Einheit
- 7; 7a, 7b, 7c: Kondensatorplatte
- 8: Spulenwindung
- 9: Leiterbahn
- 10: erste Trägerschicht
- 10a: Außenseite
- 10b: Innenseite
- 11: erster Trägerschichtbereich
- 12: zweiter Trägerschichtbereich
- 13: Klebeschicht
- 14: Zwischenschicht
- 15: Trägerstruktur
- 16: Schichtenstapel
- 17: Schichtverbund
- 18: erster Materialfilm
- 19: erste Folie
- 20: zweite Trägerschicht
- 20a: Außenseite
- 20b: Innenseite
- 21: zweiter Trägerschichtbereich
- 22: Kavität
- 25: RFID-Chip
- 26: kapazitive Brücke
- 27; 27': Kondensator
- 28: zweiter Materialfilm
- 29: zweite Folie
- 30: Verbindungsbereich
- 38: Spule
- 100: Gegenstand
- 101: Feuchtigkeitssensor
- 102: Blisterpackung
- 103: elektronisches Siegel
- *105*: versiegeltes Objekt
- F: Falt- oder Knicklinke
- G: Grundflächenbereich
- x: erste laterale Richtung
- y: zweite laterale Richtung
- z: dritte Richtung

## Patentansprüche

1. Gegenstand (100), der zumindest Folgendes aufweist:
- mindestens eine erste Leiterstruktur (1),
- zumindest eine elektronische Einheit (5),
- mindestens eine zweite Leiterstruktur (2), die von der ersten Leiterstruktur (1) und/oder von der elektronischen Einheit (5) galvanisch getrennt, aber daran elektrisch angekoppelt oder ankoppelbar ist, und
- eine Trägerstruktur (15) mit zumindest einer ersten biegsamen Trägerschicht (10), einem ersten Trägerschichtbereich (11) der ersten biegsamen Trägerschicht (10) und mit einem zweiten Trägerschichtbereich (12; 21),
- wobei die Trägerstruktur (15) in einem Grundflächenbereich (G), der zumindest einen Teil der Grundfläche der Trägerstruktur (15) umfasst, als Schichtenstapel (16) ausgebildet ist,
- wobei der Schichtenstapel (16) in dem Grundflächenbereich (G) zumindest den ersten Trägerschichtbereich (11) der ersten Trägerschicht (10) und den zweiten Trägerschichtbereich (12; 21) umfasst,
- wobei zumindest ein Teil der ersten (1) und der zweiten Leiterstruktur (2) in dem Grundflächenbereich (G) angeordnet ist,
- wobei die erste Leiterstruktur (1) und/oder die elektronische Einheit (5) an den ersten Trägerschichtbereich (11) gelötet, gebondet, auf ihn aufgeklebt oder aufgedruckt oder auf sonstige Weise mit dem ersten Trägerschichtbereich (11) verbunden und/oder in ihn eingearbeitet ist,
- wohingegen die zweite Leiterstruktur (2) an den zweiten Trägerschichtbereich (12; 21) gelötet, gebondet, auf ihn aufgeklebt oder aufgedruckt oder auf sonstige Weise mit dem zweiten Trägerschichtbereich (12; 21) verbunden oder in ihn eingearbeitet ist,
- wobei die zweite Leiterstruktur (2) durch einen außerhalb der elektronischen Einheit (5) angeordneten Flächenbereich des Schichtenstapels (16) in einem zusammengelegten Zustand der biegsamen Trägerschicht an die erste Leiterstruktur (1) elektrisch angekoppelt ist,
- wobei die erste Leiterstruktur (1) an dem ersten Trägerschichtbereich (11) und/oder an der ersten Trägerschicht (10) angeordnet ist und an die elektronische Einheit (5) oder an einzelne Bauelemente oder Teile der elektronischen Einheit (5) galvanisch angeschlossen ist, **dadurch gekennzeichnet, dass** die erste (1) und die zweite Leiterstruktur (2) in dem Grundflächenbereich (G) des Schichtenstapels (16) gemeinsam mindestens ein zur induktiven Übertragung geeignetes Paar von Spulen (28) bilden.

2. Gegenstand nach Anspruch 1, **dadurch gekennzeichnet, dass** der zweite Trägerschichtbereich (21) eine zweite biegsame Trägerschicht (20) ist oder zumindest einen Teilbereich einer anderen, zweiten biegsamen Trägerschicht (20) umfasst, wobei der erste Trägerschichtbereich (11) und der zweite Trägerschichtbereich (21) Flächenbereiche der Trägerschichten (10; 20) sind, deren Grundflächen einander zumindest bereichsweise überlappen.

3. Gegenstand nach Anspruch 1 oder 2, **dadurch gekennzeichnet, dass** die erste (1) und die zweite Leiterstruktur (2) strukturierte leitfähige Beschichtungen, etwa mit Siebdruck, Flexodruck oder einer anderen Drucktechnik erzeugte Bedruckungen der Trägerschichtbereiche (11; 12; 21) und/oder der Trägerschichten (10; 20), oder auf die Trägerschichtbereiche (11; 12; 21) und/oder Trägerschichten (10; 20) gestanzte, aufgeklebte und/oder durch einen Ätzvorgang oder anderweitigen Bearbeitungsvorgang an den Trägerschichtbereichen (11; 12; 21) und/oder Trägerschichten (10; 20) erzeugte leitfähige Strukturen sind.

4. Gegenstand nach einem der Ansprüche 1 bis 3, **dadurch gekennzeichnet, dass** die zweite Leiterstruktur (2) eine Antenne (3), ein kapazitiv beeinflussbares Sensorelement (4) eines Bedienfeldes oder eines Bedienelements, eine Kondensatorplatte (7), eine Spule (28) oder Spulenwindung (8) und/oder eine Leiterbahn (9) umfasst.

5. Gegenstand nach Anspruch 1, **dadurch gekennzeichnet, dass** die elektronische Einheit (5) außerhalb des Grundflächenbereichs (G) des Schichtenstapels (16) angeordnet ist, in dem die erste (1) und die zweite Leiterstruktur (2) elektrisch miteinander gekoppelt oder koppelbar sind.

6. Gegenstand nach einem der Ansprüche 1 bis 5, **dadurch gekennzeichnet, dass** der Schichtenstapel (16) ein Folienverbund, Schichtverbund (17) oder eine sonstige Mehrschichtstruktur ist, die eine elektrische Messung, Signalübertragung und/oder die Ausbildung, Aufrechterhaltung und/oder Überwachung eines Wechselstromkreises ermöglicht.

7. Gegenstand nach einem der Ansprüche 1 bis 6, **dadurch gekennzeichnet, dass** die erste (1) und die zweite Leiterstruktur (2) auf voneinander abgewandten Außenflächen der beiden Trägerschichtbereiche (11; 12; 21) oder Trägerschichten (10; 20) oder auf deren einander zugewandten Innenflächen angeordnet sind oder dass genau eine von beiden Leiterstrukturen (1, 2) auf derjenigen Seite des jeweiligen Trägerschichtbereichs (11; 12; 21) oder der jeweiligen Trägerschicht (10; 20) angeordnet ist, der bzw. die dem anderen Trägerschichtbereich (12; 21; 11) oder der anderen Trägerschicht (20; 10) zugewandt ist.

8. Gegenstand nach einem der Ansprüche 1 bis 3 oder 5 bis 7, **dadurch gekennzeichnet, dass** der Gegenstand (100) ein kapazitiver Feuchtigkeitssensor (101), beispielsweise ein Feuchtigkeitssensor (101) für Kleidungsstücke, Inkontinenzwindeln oder für technische Anlagen oder Geräte ist.

9. Gegenstand nach einem der Ansprüche 1 bis 3 oder 5 bis 7, **dadurch gekennzeichnet, dass** der Gegenstand (100) eine Blisterpackung (102) ist, bei der beim Entnehmen einer Tablette oder Kapsel eine zugeordnete erste oder zweite Leiterstruktur (1; 2) zumindest lokal durchtrennt wird.

10. Gegenstand nach einem der Ansprüche 1 bis 9, **dadurch gekennzeichnet, dass** die erste (11) und/oder die zweite Leiterstruktur (2) eine Mobilfunkantenne oder eine Antenne (3) für NFC-Signale, Bluetooth, WLAN oder andere Funksysteme umfasst und/oder dass die elektronische Einheit (5) einen RFID-Transponder oder einen RFID-Chip (25) umfasst.

11. Gegenstand nach einem der Ansprüche 1 bis 10, **dadurch gekennzeichnet, dass** die erste Leiterstruktur (1) zwei Kondensatorplatten (7a, 7b) umfasst und dass die zweite Leiterstruktur (2) eine kapazitive Brücke (26) zwischen den beiden Kondensatorplatten (7a, 7b) der ersten Leiterstruktur (1) ist.

12. Gegenstand nach einem der Ansprüche 1 oder 3 bis 11, **dadurch gekennzeichnet, dass** der zweite Trägerschichtbereich (12) ein weiterer Trägerschichtbereich der ersten Trägerschicht (10) ist, der in den Schichtenstapel (16) integriert ist, wobei die erste Trägerschicht (10) zumindest stellenweise umgebogen, geknickt, gefaltet und/oder auf sonstige Weise zu einer geschlossenen Schleife, Schlaufe oder zu einem Band oder Streifen geformt ist.

13. Gegenstand nach Anspruch 12, **dadurch gekennzeichnet, dass** der Gegenstand (100) ein elektronisches Siegel (103) ist und dass die erste Trägerschicht (10) einen von dem ersten Trägerschichtbereich (11) bis zum zweiten Trägerschichtbereich (12) führenden Verbindungsbereich (30) aufweist und dass zwischen die erste Leiterstruktur (1) und die zweite Leiterstruktur (2) zumindest die elektronische Einheit (5) zwischengeschaltet ist.

14. Gegenstand (100), der zumindest Folgendes aufweist:
- mindestens eine erste Leiterstruktur (1),
- mindestens eine zweite Leiterstruktur (2),
- zumindest eine elektronische Einheit (5), die zwischen die erste (1) und die zweite Leiterstruktur (2) zwischengeschaltet ist, und
- eine Trägerstruktur (15) mit zumindest einer biegsamen Trägerschicht (10),
- wobei die Trägerstruktur (15) zumindest einen ersten Trägerschichtbereich (11), einen zweiten Trägerschichtbereich (12) und einen Verbindungsbereich (30), der den ersten Trägerschichtbereich (11) mit dem zweiten Trägerschichtbereich (12) verbindet, aufweist,
- wobei die Trägerstruktur (15) in der Weise faltbar, knickbar, umbiegbar oder anderweitig zusammenlegbar ist, dass
- der erste Trägerschichtbereich (11) und der zweite Trägerschichtbereich (12) zu einem Schichtenstapel (16) gestapelt werden und dass
- in einem außerhalb der elektronischen Einheit (5) angeordneten Flächenbereich des Schichtenstapels (16) ein Endstück oder Teilstück der ersten Leiterstruktur (1), das im ersten Trägerschichtbereich (11) angeordnet ist, mit einem Endstück oder Teilstück der zweiten Leiterstruktur (2), das im zweiten Trägerschichtbereich (12) angeordnet ist, in einem zusammengelegten Zustand der biegsamen Trägerschicht elektrisch gekoppelt wird, **dadurch gekennzeichnet, dass** die Endstücke oder Teilstücke der ersten (1) und der zweite Leiterstruktur (2) gemeinsam mindestens ein zur induktiven Übertragung geeignetes Paar von Spulen (28) bilden.

15. Gegenstand nach Anspruch 14, **dadurch gekennzeichnet, dass** der Gegenstand ein elektronisches Siegel (103) ist, dessen erster (11) und zweiter Trägerschichtbereich (12) aneinander heranführbar und/oder aneinander befestigbar sind, um einen Wechselstromkreis zu schließen.

16. Verfahren zur Herstellung eines Gegenstands (100), der eine elektronische Einheit und Leiterstrukturen auf einer Trägerstruktur aufweist, wobei das Verfahren zumindest Folgendes umfasst:
a) Aufbringen mindestens einer ersten Leiterstruktur (1) und zumindest einer elektronischen Einheit (5) auf eine erste biegsame Trägerschicht (10) sowie Aufbringen mindestens einer zweiten Leiterstruktur (2) auf eine zweite biegsame Trägerschicht (20) und
b) Fertigstellen des Gegenstandes (100) durch Verbinden, insbesondere Verkleben oder Verschweißen der ersten biegsamen Trägerschicht (10) mit der zweiten biegsamen Trägerschicht (20), wodurch
- eine Trägerstruktur (15) gebildet wird, die in einem Grundflächenbereich (G), der zumindest eine Teilfläche der Grundfläche der Trägerstruktur (15) umfasst, als Schichtenstapel (16) ausgebildet ist und wodurch
- in einem Flächenbereich des Schichtenstapels (16), der außerhalb der elektronischen Einheit (5) angeordnet ist, in einem zusammengelegten Zustand der biegsamen Trägerschicht die zweite Leiterstruktur (2) an die erste Leiterstruktur (1) elektrisch angekoppelt wird, sodass die Endstücke oder Teilstücke der ersten (1) und der zweite Leiterstruktur (2) gemeinsam mindestens ein zur induktiven Übertragung geeignetes Paar von Spulen (28) bilden.

## Claims

1. An object (100), comprising at least the following:
- at least one first conductor structure (1),
- at least one electronic unit (5),
- at least one second conductor structure (2), which is galvanically isolated from the first conductor structure (1) and/or from the electronic unit (5) but is coupled or can be coupled electrically thereto, and
- a carrier structure (15) with at least one first pliable carrier layer (10), a first carrier layer region (11) of the first pliable carrier layer (10) and with a second carrier layer region (12; 21),
- wherein the carrier structure (15) is formed as a layer stack (16) in a base surface region (G), which comprises at least one part of the base surface of the carrier structure (15),
- wherein the layer stack (16) in the base surface region (G) comprises at least the first carrier layer region (11) of the first carrier layer (10) and the second carrier layer region (12; 21),
- wherein at least one part of the first (1) and of the second conductor structure (2) is disposed in the base surface region (G),
- wherein the first conductor structure (1) and/or the electronic unit (5) is soldered, bonded to the first carrier layer region (11), adhered or printed on it or joined in some other way with the first carrier layer region (11) and/or worked into it,
- while the second conductor structure (2) is soldered, bonded to the second carrier layer region (12; 21), adhered or printed on it or joined in some other way with the second carrier layer region (12; 21) or worked into it,
- wherein the second conductor structure (2) is coupled electrically to the first conductor structure (1) in a folded state of the pliable carrier layer (10) by a surface region of the layer stack (16) disposed outside the electronic unit (5),
- wherein the first conductor structure (1) is arranged on the first carrier layer region (11) and/or on the first carrier layer (10) and is galvanically connected to the electronic unit (5) or to individual components or parts of the electronic unit (5), **characterized in that** the first (1) and second conductor structures (2) together form at least a pair of coils (28) suitable for inductive transmission in the base surface region (G) of the layer stack (16).

2. Object according to claim 1, **characterized in that** the second carrier layer region (21) comprises a second pliable carrier layer (20) or at least one partial region of another, second carrier pliable layer (20), wherein the first carrier layer region (11) and the second carrier layer region (21) are surface regions of the carrier layers (10; 20), the base surfaces of which overlap one another at least regionally.

3. Object according to claim 1 or 2, **characterized in that** the first (1) and the second conductor structure (2) are structured conductive coatings, prints of the carrier layer regions (11; 12; 21) and/or of the carrier layers (10; 20) created, for instance with screen printing, flexography or any other printing technique, or conductive structures stamped, adhered onto the carrier layer regions (11; 12; 21) and/or carrier layers (10;20) and/or created by an etching process or other kind of machining process on the carrier layer regions (11; 12; 21) and/or carrier layers (10;20).

4. Object according to one of claims 1 to 3, **characterized in that** the second conductor structure (2) comprises an antenna (3), a sensor element (4), which can be capacitively influenced, of a control panel or of a control element, a capacitor plate (7), an inductor (28) or induction coil (8) and/or a conductor track (9).

5. Object according to claim 1, **characterized in that** the electronic unit (5) is arranged outside the base surface region (G) of the layer stack (16), where the first (1) and the second conductor structures (2) are coupled or can be coupled to one another electrically.

6. Object according to one of claims 1 to 5, **characterized in that** the layer stack (16) is a foil composite, layer composite (17) or some other multilayer structure, which permits an electrical measurement, signal transmission and/or the formation, maintenance and/or monitoring of an alternating current circuit.

7. Object according to one of claims 1 to 6, **characterized in that** the first (1) and the second conductor structure (2) are disposed on outer surfaces, facing away from one another, of the two carrier layer regions (11; 12; 21) or carrier layers (10; 20) or on their inner surfaces, facing one another, or **in that** precisely one of the two conductor structures (1, 2) is disposed on that side of the respective carrier layer regions (11; 12; 21) or of the respective carrier layer (10; 20) which is facing the other carrier layer region or regions (12; 21; 11) or the other carrier layer (20; 10).

8. Object according to one of claims 1 to 3 or 5 to 7, **characterized in that** the object (100) is a capacitive moisture sensor (101), for example a moisture sensor (101) for clothing items, incontinence diapers or for technical systems or instruments.

9. Object according to one of claims 1 to 3 or 5 to 7, **characterized in that** the object (100) is a blister pack (102), in which an associated first or second conductor structure (1; 2) is severed at least locally during the removal of a tablet or capsule.

10. Object according to one of claims 1 to 9, **characterized in that** the first (11) and/or the second conductor structure (2) comprises a mobile communications antenna or an antenna (3) for NFC signals, Bluetooth, WLAN or other radio systems and/or **in that** the electronic unit (5) comprises an RFID transponder or an RFID chip (25).

11. Object according to one of claims 1 to 10, **characterized in that** the first conductor structure (1) comprises two capacitor plates (7a, 7b) and **in that** the second conductor structure (2) is a capacitive bridge (26) between the two capacitor plates (7a, 7b) of the first conductor structure (1).

12. Object according to one of claims 1 or 3 to 11, **characterized in that** the second carrier layer region (12) is a further carrier layer region of the first carrier layer (10), which is integrated into the layer stack (16), wherein the first carrier layer (10), at least in places, is bent back, creased, folded and/or formed in some other way to a closed loop, strap or to a ribbon or strip.

13. Object according to claim 12, **characterized in that** the object (100) is an electronic seal (103) and **in that** the first carrier layer (10) has a joint region (30) extending from the first carrier layer region (11) to the second carrier layer region (12) and **in that** at least the electronic unit (5) is interconnected between the first conductor structure (1) and the second conductor structure (2).

14. Object (100), comprising at least the following:
- at least one first conductor structure (1),
- at least one second conductor structure (2),
- at least one electronic unit (5), which is interconnected between the first (1) and the second conductor structure (2), and
- a carrier structure (15) with at least one pliable carrier layer (10),
- wherein the carrier structure (15) has at least one first carrier layer region (11), one second carrier layer region (12) and one joint region (30), which joins the first carrier layer region (11) to the second carrier layer region (12),
- wherein the carrier structure (15) can be folded, creased, bent back or otherwise placed together, such that the first carrier layer region (11) and the second carrier layer region (12) are stacked as a layer stack (16), and that,
in a surface region of the layer stack (16) disposed outside the electronic unit (5) an end piece or partial piece of the first conductor structure (1), which is disposed in the first carrier layer region (11), gets coupled electrically to an end piece or partial piece of the second conductor structure (2) disposed in the second carrier layer region (12) in a folded state of the pliable carrier layer, **characterized in that** the end pieces or partial pieces of the first (1) and second conductor structures (2) together form at least a pair of coils (28) suitable for inductive transmission.

15. Object according to claim 14, **characterized in that** the object is an electronic seal (103), the first (11) and second carrier layer region (12) of which can be guided up to one another and/or fastened to one another, in order to close an alternating current circuit.

16. Method for manufacturing an object (100), which has an electronic unit and conductor structures on a carrier structure, wherein the method comprises at least the following:
a) application of at least one first conductor structure (1) and at least one electronic unit (5) on a first pliable carrier layer (10) as well as application of at least one second conductor structure (2) on a pliable second carrier layer (20), and
b) finishing of the object (100) by joining, especially adhering or welding, of the first pliable carrier layer (10) with the pliable second carrier layer (20), whereby
- a carrier structure (15) is formed which, in a base surface region (G) that comprises the at least one partial surface of the base surface of the carrier structure (15), is constructed as a layer stack (16), and whereby
- in an area of the layer stack (16) located outside the electronic unit (5), in a folded state of the pliable carrier layer the second conductor structure (2) gets electrically coupled to the first conductor structure (1), such that the end pieces or partial pieces of the first (1) and second conductor structures (2) together form at least a pair of coils (28) suitable for inductive transmission.

## Revendications

1. Objet (100) comprenant au moins :
- au moins une première structure conductrice (1),
- au moins une unité électronique (5),
- au moins une deuxième structure conductrice (2) séparée galvaniquement de la première structure conductrice (1) et/ou de l'unité électronique (5), mais couplée ou pouvant être couplée électriquement à celles-ci, et
- une structure de support (15) ayant au moins une première couche de support flexible (10), une première zone de couche de support (11) de la première couche de support flexible (10) et une deuxième zone de couche de support (12 ; 21),
- la structure de support (15) étant réalisée sous forme d'un empilement de couches (16) dans une zone de surface de base (G) qui comprend au moins une partie de la surface de base de la structure de support (15),
- l'empilement de couches (16) dans la zone de surface de base (G) comprenant au moins la première zone de couche de support (11) de la première couche de support (10) et la deuxième zone de couche de support (12 ; 21),
- au moins une partie de la première structure conductrice (1) et de la deuxième structure conductrice (2) étant disposée dans la zone de surface de base (G),
- la première structure conductrice (1) et/ou l'unité électronique (5) étant soudée, fixée par bonding, collée, imprimée ou reliée autrement à la première zone de couche de support (11) et/ou intégrée dans celle-ci,
- tandis que la deuxième structure conductrice (2) est soudée, fixée par bonding, collée, imprimée ou reliée autrement à la deuxième zone de couche de support (12 ; 21) ou intégrée dans celle-ci,
- la deuxième structure conductrice (2) étant couplée électriquement à la première structure conductrice (1) par une zone de surface de l'empilement de couches (16) disposée à l'extérieur de l'unité électronique (5) dans un état plié de la couche de support flexible,
- la première structure conductrice (1) étant disposée sur la première zone de couche de support (11) et/ou sur la première couche de support (10) et étant reliée galvaniquement à l'unité électronique (5) ou à des composants individuels ou à des parties de l'unité électronique (5), **caractérisé en ce que** la première structure conductrice (1) et la deuxième structure conductrice (2) forment ensemble, dans la zone de surface de base (G) de l'empilement de couches (16), au moins une paire de bobines (28) adaptée à la transmission inductive.

2. L'objet selon la revendication 1, **caractérisé en ce que** la deuxième zone de couche de support (21) est une deuxième couche de support flexible (20) ou comprend au moins une section d'une autre deuxième couche de support flexible (20), la première zone de couche de support (11) et la deuxième zone de couche de support (21) étant des zones de surface des couches de support (10 ; 20) dont les surfaces de base se chevauchent au moins partiellement.

3. L'objet selon la revendication 1 ou 2, **caractérisé en ce que** la première structure conductrice (1) et la deuxième structure conductrice (2) sont des revêtements conducteurs structurés, par exemple des impressions réalisées par sérigraphie, flexographie ou une autre technique d'impression, sur les zones de couche de support (11 ; 12 ; 21) et/ou sur des couches de support (10 ; 20), ou sont des structures conductrices estampées, collées et/ou produites par un processus de gravure ou un autre processus d'usinage sur les zones de couche de support (11 ; 12 ; 21) et/ou sur les couches de support (10 ; 20).

4. L'objet selon l'une des revendications 1 à 3, **caractérisé en ce que** la deuxième structure conductrice (2) comprend une antenne (3), un élément capteur (4) pouvant être influencé de manière capacitive, un panneau de commande ou un élément de commande, une plaque de condensateur (7), une bobine (28) ou un enroulement de bobine (8) et/ou une piste conductrice (9).

5. L'objet selon la revendication 1, **caractérisé en ce que** l'unité électronique (5) est disposée à l'extérieur de la zone de surface de base (G) de l'empilement de couches (16), dans laquelle la première structure conductrice (1) et la deuxième structure conductrice (2) sont couplées ou peuvent être couplées électriquement l'une à l'autre.

6. L'objet selon l'une des revendications 1 à 5, **caractérisé en ce que** l'empilement de couches (16) est un composite de feuilles, un composite de couches (17) ou une autre structure multicouche qui permet une mesure électrique, une transmission de signaux et/ou la formation, la continuité et/ou la surveillance d'un circuit à courant alternatif.

7. L'objet selon l'une des revendications 1 à 6, **caractérisé en ce que** la première structure conductrice (1) et la deuxième structure conductrice (2) sont disposées sur des surfaces extérieures opposées des deux zones de couche de support (11 ; 12 ; 21) ou des couches de support (10 ; 20) ou sur leurs surfaces intérieures opposées, ou **en ce qu'**exactement l'une des deux structures conductrices (1, 2) est disposée sur ce côté de la zone de couche de support respective (11 ; 12 ; 21) ou de la couche de support respective (10 ; 20) qui est tournée vers l'autre zone de couche de support (12 ; 21 ; 11) ou l'autre couche de support (20 ; 10).

8. L'objet selon l'une des revendications 1 à 3 ou 5 à 7, **caractérisé en ce que** l'objet (100) est un capteur d'humidité capacitif (101), tel que par exemple un capteur d'humidité (101) pour vêtements, couches pour incontinence ou pour installations ou appareils techniques.

9. L'objet selon l'une des revendications 1 à 3 ou 5 à 7, **caractérisé en ce que** l'objet (100) est un emballage blister (102) dans lequel, lors du retrait d'un comprimé ou d'une capsule, une première ou une deuxième structure conductrice (1 ; 2) associée est sectionnée au moins localement.

10. L'objet selon l'une des revendications 1 à 9, **caractérisé en ce que** la première structure conductrice (11) et/ou la deuxième structure conductrice (2) comprend une antenne de téléphonie mobile ou une antenne (3) pour les signaux NFC, Bluetooth, WLAN ou d'autres systèmes radio, et/ou **en ce que** l'unité électronique (5) comprend un transpondeur RFID ou une puce RFID (25).

11. L'objet selon l'une des revendications 1 à 10, **caractérisé en ce que** la première structure conductrice (1) comprend deux plaques de condensateur (7a, 7b), et **en ce que** la deuxième structure conductrice (2) est un pont capacitif (26) entre les deux plaques de condensateur (7a, 7b) de la première structure conductrice (1).

12. L'objet selon l'une des revendications 1 ou 3 à 11, **caractérisé en ce que** la deuxième zone de couche de support (12) est une autre zone de couche de support de la première couche de support (10) qui est intégrée dans l'empilement de couches (16), la première couche de support (10) étant au moins par endroits courbée, pliée, plissée et/ou formée autrement en une boucle fermée, une dragonne, un ruban ou une bande.

13. L'objet selon la revendication 12, **caractérisé en ce que** l'objet (100) est un sceau électronique (103), **en ce que** la première couche de support (10) présente une zone de liaison (30) menant de la première zone de couche de support (11) à la deuxième zone de couche de support (12) et **en ce qu'**au moins l'unité électronique (5) est intercalée entre la première structure conductrice (1) et la deuxième structure conductrice (2).

14. Objet (100) comprenant au moins :
- au moins une première structure conductrice (1),
- au moins une deuxième structure conductrice (2),
- au moins une unité électronique (5) intercalée entre la première structure conductrice (1) et la deuxième structure conductrice (2), et
- une structure de support (15) ayant au moins une couche de support flexible (10),
- la structure de support (15) comprenant au moins une première zone de couche de support (11), une deuxième zone de couche de support (12) et une zone de liaison (30) reliant la première zone de couche de support (11) à la deuxième zone de couche de support (12),
- la structure de support (15) pouvant être plissée, pliée, courbée ou autrement repliée de telle sorte que
- la première zone de couche de support (11) et la deuxième zone de couche de support (12) soient empilées pour former un empilement de couches (16) et que
- dans une zone de surface de l'empilement de couches (16) disposée à l'extérieur de l'unité électronique (5), une extrémité ou une partie de la première structure conductrice (1), disposée dans la première zone de couche de support (11), soit couplée électriquement à une extrémité ou une partie de la deuxième structure conductrice (2), disposée dans la deuxième zone de couche de support (12), dans un état replié de la couche de support flexible, **caractérisé en ce que** les extrémités ou parties de la première structure conductrice (1) et de la deuxième structure conductrice (2) forment ensemble au moins une paire de bobines (28) adaptée à la transmission inductive.

15. L'objet selon la revendication 14, **caractérisé en ce que** l'objet est un sceau électronique (103) dont la première zone de couche de support (11) et la deuxième zone de couche de support (12) peuvent être rapprochées et/ou fixées l'une à l'autre afin de fermer un circuit électrique alternatif.

16. Procédé de fabrication d'un objet (100) comprenant une unité électronique et des structures conductrices sur une structure de support, le procédé comprenant au moins :
a) l'application d'au moins une première structure conductrice (1) et d'au moins une unité électronique (5) sur une première couche de support flexible (10), ainsi que l'application d'au moins une deuxième structure conductrice (2) sur une deuxième couche de support flexible (20), et
b) la finition de l'objet (100) par assemblage, en particulier par collage ou soudage, de la première couche de support flexible (10) avec la deuxième couche de support flexible (20), par quoi
- une structure de support (15) est formée, conçue comme un empilement de couches (16) dans une zone de surface de base (G) comprenant au moins une surface partielle de la surface de base de la structure de support (15), et par quoi
- dans une zone de surface de l'empilement de couches (16) disposée à l'extérieur de l'unité électronique (5), dans un état replié de la couche de support flexible, la deuxième structure conductrice (2) est couplée électriquement à la première structure conductrice (1), de sorte que les extrémités ou les parties de la première structure conductrice (1) et de la deuxième structure conductrice (2) forment ensemble au moins une paire de bobines (28) adaptées à la transmission inductive.
